(19) Europäisches Patentamt
European Patent Office
Office européen des brevets

(11) Veröffentlichungsnummer: **0 096 189**
**A2**

(12) # EUROPÄISCHE PATENTANMELDUNG

(21) Anmeldenummer: **83103610.8**

(22) Anmeldetag: **14.04.83**

(51) Int. Cl.³: **A 01 N 43/70**
//C07D251/52

(30) Priorität: **11.06.82 DE 3222147**

(43) Veröffentlichungstag der Anmeldung:
**21.12.83 Patentblatt 83/51**

(84) Benannte Vertragsstaaten:
**AT BE CH DE FR GB IT LI LU NL SE**

(71) Anmelder: **CHEMISCHE WERKE HÜLS AG**
**- RSP Patente / PB 15 - Postfach 13 20**
**D-4370 Marl 1(DE)**

(72) Erfinder: **Baltruschat, Helmut, Dr.**
**Am Hagenbach 3**
**D-4405 Nottuln-Darup(DE)**

(72) Erfinder: **Schnurbusch, Horst, Dr.**
**Overwegstrasse 36**
**D-4690 Herne 1(DE)**

(74) Vertreter: **Steil, Hanna, Dipl.-Chem.**
**RSP PATENTE - PB 15 Postfach 1320**
**D-4370 Marl 1(DE)**

(54) **Verwendung gegebenenfalls substituierter Thiomethyl-cycloalkyl-amino-s-triazine als selektive Mittel gegen Unkräuter und Schadgräser.**

(57) Zahlreiche zweikeimblättrige Unkräuter, sowie Ackerfuchsschwanz und vor allem Hirsearten lassen sich bei guter Selektivität in Gerste, Weizen, Reis, sowie in Baumwolle oder Soja erfolgreich bekämpfen mit Cyclopentylamino-oder Cyclohexylamino-methylthio-s-triazinen der allgemeinen Formel

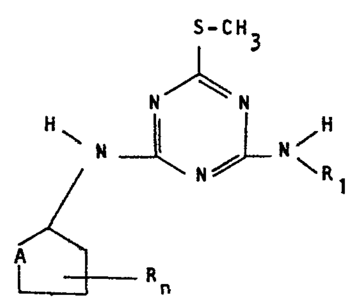

in der

$R_1$ einen gerad- oder verzweigtkettigen Alkyl-, Alkoxy-, Alkoxyalkyl- oder Alkylol-Rest oder ein H-Atom

R einen Rest aus der Gruppe der Alkyle mit 1 - 3 C-Atomen und

A = $-CH_2$ oder $-CH_2-CH_2-$ bedeuten und

n für A = $-CH_2-$ jeweils 0 oder 1, 2, 3, 4, 5 oder 6, bzw. für A = $-CH_2-CH_2-$ jeweils 3, 4, 5 oder 6 sein können und wobei die Substituenten R an den Cycloalkylringen gleich oder verschieden sein können.

EP 0 096 189 A2

Croydon Printing Company Ltd

Verwendung gegebenenfalls substituierter Thiomethyl-cycloalkylamino-s-triazine als selektive Mittel gegen Unkräuter und Schadgräser

Von den 6-Thiomethyl-s-triazinen sind zahlreiche Derivate bekannt geworden, die in 2- und 4-Stellung mit primären Alkylaminen substituiert sind, z.B.

Desmetryn (2-Methylamino-4-isopropylamino)
Simetryn (2,4-bisethylamino)
Ametryn (2-Ethylamino-4-isopropylamino)
Prometryn (2,4-bisisopropylamino)
Methoprotryn (2-Isopropylamino-4(3-methoxypropylamino)
MPMT (2,4-Bis-(3-methoxypropylamino)) und
als wohl bekannteste Verbindung dieser Reihe
Terbutryn (2-Ethylamino-4-tert.butylamino).

Von den 6-Ethylthio-Derivaten ist das Dipropetryn (2,4-Bis-isopropylamino) zu nennen (vgl. R. WEGLER "Chemie der Pflanzenschutz- und Schädlingsbekämpfungsmittel", Springer Verlag, Berlin, Heidelberg, New York, Bd. 2 (1970, Seite 372-74) und Bd. 5 (1977, Seite 346 - 50)). Die meisten dieser Verbindungen sind als Boden- und Blattherbizide gegen Mono- und Dikotyledonen im Vor- und/oder Nachauflauf verwendbar. Beispielsweise ist Terbutryn in Wintergetreidearten selektiv, jedoch gegen Schadgräser wie Ackerfuchsschwanz und Windhalm nicht immer ausreichend wirksam. Auch bei den anderen 6-Methylthio-1,3,5-triazinen mit Substituenten aus der Gruppe der kurzen Alkylamine findet man ebenfalls gewöhnlich gute herbizide

Eigenschaften, jedoch läßt die selektive herbizide Wirksamkeit ebenfalls in manchen Fällen zu wünschen übrig.

2-Cyclohexylamino-Derivate der 6-Methylthio-s-triazine wurden in den Substanzen 12 bis 21 der Tabelle auf Seite 3 der JA-OS 47-23 436 vorgestellt und in der Substanz 22 ein entsprechendes Propylthio-Derivat, sowie in den Substanzen 23 bis 27 (Seite 3/4) weitere 6- Butylthio-s-triazine.
Auch bei diesen Wirkstoffen war die Selektivität gegen Nutzpflanzen und die Herbizidwirkung gegen Schadpflanzen nicht voll befriedigend.

Überraschenderweise wurde gefunden, daß bei den Alkylthio-s-triazinen die Verbindungen, die sowohl mit einem gerad- oder verzweigtkettigen relativ kurzen Amin als auch mit einem reinen oder gegebenenfalls mono-, di-, tri- oder tetraalkylierten cycloaliphatischen Amin substituiert sind, - bei gleichwertiger und zum Teil erweiterter Möglichkeit zum Einsatz als selektives Unkrautbekämpfungsmittel - eine bessere Schadgräser-Wirkung zeigen als die bisher bekannten Verbindungen.

Gegenstand der Erfindung ist also die Verwendung von Methylthio-cyclopentylamin- bzw. Methylthio-cyclohexylamin-s-triazinen der allgemeinen Formel

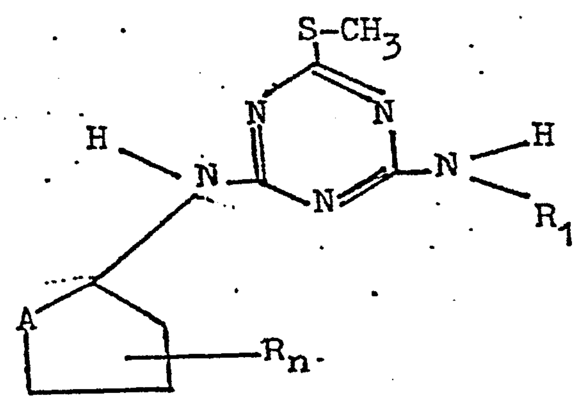

I,

in der

$R_1$ einen gerad- oder verzweigtkettigen Alkyl-, Alkoxy-, Alkoxyalkyl- oder Alkylol-Rest oder ein H-Atom,

R einen Rest aus der Gruppe der Alkyle mit ein bis drei C-Atomen und

A = $-CH_2-$ oder $-CH_2-CH_2-$ bedeuten und

n für A = $-CH_2-$ jeweils 0 oder 1,2,3,4,5 oder 6, bzw. für A = $-CH_2-CH_2-$ jeweils 3,4,5 oder 6 sein können und wobei die Substituenten R an den Cycloalkylringen gleich oder verschieden sein können,

in geeigneter Applikationsform zur selektiven Behandlung von Nutzpflanzenkulturen mit breitem Wirkungssepektrum bei Unkräutern und Schadgräsern

vor und/oder nach dem Auflaufen der Saat.

Die Ketten, die von $R_1$ gebildet werden, sollten - bei Nichtberücksichtigung der H-Atome und unabhängig vom Verzeigungsgrad - nicht mehr als 15 C- (bzw. O) Atome aufweisen.

Herstellung der erfindungsgemäßen Triazine

Die neuen Verbindungen der Formel I können nach an sich bekannten Methoden hergestellt werden, indem man Cyanurchlorid - vorzugsweise in einem Lösungsmittel oder Lösungsmittelgemisch, z.B. in Aceton, Aceton/Wasser, Dioxan, Dioxan/Wasser, Tetrahydrofuran oder in Dimethylformamid -

mit den entsprechenden Aminen bei der jeweils zweckmäßigen Temperatur, die gewöhnlich zwischen -30 °C und +50 °C liegt, umsetzt.

Zur selektiven Substitution am Triazingerüst arbeitet man nach den für die stufenweise Substitution in 1,3,5-Triazinen bekannten Arbeitsweise.

Die zuerst erhaltenen Chlor-Derivate werden in Isopropanol oder anderen Lösungsmitteln mit Natriumthiomethyl zum entsprechenden Thiomethylderivat umgesetzt.

<u>Beispiel 1</u>

<u>Darstellung von 2-Cyclopentylamino-4-amino-6-methylthio-s-triazin</u>

184,5 g (1,00 Mol) Cyanurchlorid werden in 900 ml Aceton gelöst und unter starkem Rühren im Laufe einer 1/2 Stunde in 1100 ml Wasser von 0 - 2 °C eingetropft. In die erhaltene Suspension werden dann in 30 min bei 0 - 2 °C 85,0 g (1,00 Mol) Cyclopentylamin, ebenfalls unter starkem Rühren, eingetropft. Der Ansatz wird 30 min nachgerührt, wobei das suspendierte Produkt fast vollständig in Lösung geht.

Zur Neutralisation der abgespaltenen HCl werden anschließend 40,0 g (1,00 Mol) NaOH als 30 %ige Lösung in 30 min zugetropft. Dabei wird in den ersten 20 - 25 Min. im allgemeinen der pH-Wert 8,0 nicht überschritten. Erst gegen Ende der Neutralisation wird ein pH von 8,5 - 9,0 erreicht.

Der Ansatz wird dabei stark gerührt und die Temperatur bei 0 - 2 °C gehalten. Während der NaOH-Zugabe fällt das Dichlor-cyclopentylamin-s-Triazin feinverteilt aus. Es liegt dann als gut rührbare Suspension vor.

Anschließend werden unter Rühren 70,0 g (1,03 Mol) 25 %iges $NH_4OH$ bei 5 °C zugegeben. Der Ansatz wird auf 30 - 35 °C erwärmt und 4 h nachgerührt. Die folgende Neutralisation der abgespaltenen HCl mit 1,00 Mol 30 %iger NaOH erfordert weitere 8 h Zeit.

Danach wird das Aceton aus dem Ansatz abdestilliert, wobei das 2-Chlor-4-cyclopentylamino-6-amino-s-triazin als weiches, körniges Produkt ausfällt und abfiltiert wird.

Zur weiteren Umsetzung werden 213,5 g (1,00 Mol) davon mit 336 g (1,20 Mol) einer 25 %igen wässrigen $NaSCH_3$-Lösung und 350 ml Isopropanol zusammengegeben und 15 h bei 82 - 83 °C gerührt. Das Produkt bleibt klar gelöst, aus der organischen Phase trennt sich eine wässrige Schicht ab.

Zur Aufarbeitung wird Isopropylalkohol abdestilliert und das ausgefallene gelbe harzartige Produkt dreimal mit 80 °C heißem Wasser gewaschen. Nach dem Trocknen im Vakuum bei 80 °C beträgt die Ausbeute 70 %, bezogen auf eingesetztes Cyanurchlorid.

Beispiel 2

Darstellung des 2-Cyclopentylamino-4-methylamino-6-methylthio-s-triazin

Die Herstellung des 2-Cyclopentylamino-4,6-Dichlor-s-triazin erfolgt, wie in den ersten beiden Verfahrensschritten von Beispiel 1 (bis Seite 5, Zeile 5) beschrieben, wurde.
Anschließend werden in 45 min bei 0 - 5 °C 31,9 g (1,03 Mol) Methylamin als 30 %ige wässrige Lösung unter Rühren eingetropft und 2 h gerührt, wobei sich die Temperatur langsam auf 30 - 35 °C erhöht. Danach wird noch 4 h bei 30 - 35 °C gerührt. Die anschließende Neutralisation mit 1,00 Mol 30 %iger NaOH dauert etwa 4 h.

Zur Abtrennung des 2-Chlor-4-cyclopentylamino-6-methylamino-s-triazins wird das Aceton abdestilliert und das ausgefallene weiße Produkt abgesaugt.

Danach wird mit 227,5 g (1,00 Mol) des Zwischenproduktes genauso verfahren, wie es bei Beispiel 1 (in den Zeilen 19 bis 24 von Seite 5) beschrieben ist.

Nach dem Trocknen im Vakuum bei 80 °C beträgt die Ausbeute von 2-Cyclopentylamino-4-methylamino-6-methylthio-s-triazin 75 %, bezogen auf eingesetztes Cyanurchlorid.

In analoger Weise lassen sich andere Glieder der erfindungsgemäßen Cycloalkylamino-s-triazine herstellen. Eine Auswahl davon ist - einschließlich der Beispiele 1 und 2 - in Tabelle 1 a, (S.8 und 9 ) angeführt.

Darin bedeuten in den Beispielen 15 bis 26:
TMCP = 2,2,4- bzw. 2,4,4-Trimethylcyclopentyl-

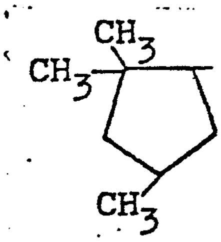

oder

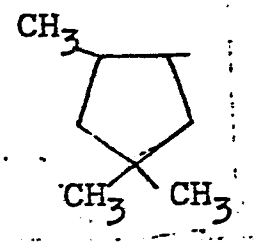

und

in den Beispielen 27 bis 36:
TMC = 3,3,5-Trimethylcyclohexyl-

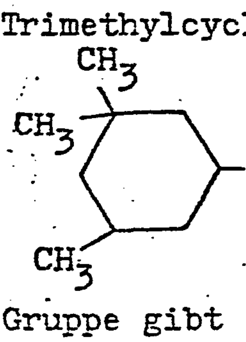

(Bei dieser Gruppe gibt es stabile cis- und trans-Formen und (cis-/trans-) Gemische).

Die Tabelle 1 b (Seite 10 bis 12) gibt die Bedeutung von $R_1$, A und n, sowie die Stellung am Ring, die durch die n-Substituenten R ($=CH_3$) besetzt sind, bei diesen 36 Verbindungen in Bezug auf die Formel I an, sowie ihre Schmelzpunkte.

Tabelle 1 a Liste der hergestellten Methylthiocycloalkyl-s-triazine

| | | | |
|---|---|---|---|
| Beispiel | 1 | = | 2-Cyclopentylamino-4-amino-6-methylthio-s-triazin |
| Beispiel | 2 | = | 2-Cyclopentylamino-4-methylamino-6-methylthio-s-triazin |
| Beispiel | 3 | = | 2-Cyclopentylamino-4-ethylamino-6-methylthio-s-triazin |
| Beispiel | 4 | = | 2-Cyclopentylamino-4-isopropylamino-6-methylthio-s-triazin |
| Beispiel | 5 | = | 2-Cyclopentylamino-4-propylamino-6-methylthio-s-triazin |
| Beispiel | 6 | = | 2-Cyclopentylamino-4-tert.-butylamino-6-methylthio-s-triazin |
| Beispiel | 7 | = | 2-Cyclopentylamino-4-2'hydroxyethylamino-6-methylthio-s-triazin |
| Beispiel | 8 | = | 2-Cyclopentylamino-4-3'methoxy-propylamino-6-methylthio-s-triazin |
| Beispiel | 9 | = | 2-Cyclopentylamino-4-3'ethoxy-propylamino-6-methylthio-s-triazin |
| Beispiel | 10 | = | 2-Cyclopentylamino-4-2'methoxy-ethylamino-6-methylthio-s-triazin |
| Beispiel | 11 | = | 2-Cyclopentylamino-4-3'butoxy-propylamino-6-methylthio-s-triazin |
| Beispiel | 12 | = | 2-Cyclopentylamino-4-3'hexoxy-propylamino-6-methylthio-s-triazin |
| Beispiel | 13 | = | 2-Cyclopentylamino-4-3'isopropoxy-propylamino-6-methylthio-s-triazi |
| Beispiel | 14 | = | 2-Cyclopentylamino-4-3'sec.-butoxy-propylamino-6-methylthio-s-tria: |
| Beispiel | 15 | = | 2-TMCPamino-4-amino-6-methylthio-s-triazin |
| Beispiel | 16 | = | 2-TMCPamino-4-methylamino-6-methylthio-s-triazin |
| Beispiel | 17 | = | 2-TMCPamino-4-ethylamino-6-methylthio-s-triazin |
| Beispiel | 18 | = | 2-TMCPamino-4-isopropylamino-6-methylthio-s-triazin |
| Beispiel | 19 | = | 2-TMCPamino-4-propylamino-6-methylthio-s-triazin |
| Beispiel | 20 | = | 2-TMCPamino-4-tert.-butylamino-6-methylthio-s-triazin |
| Beispiel | 21 | = | 2-TMCPamino-4-2'methoxy-ethylamino-6-methylthio-s-triazin |
| Beispiel | 22 | = | 2-TMCPamino-4-3'methoxy-propylamino-6-methylthio-s-triazin |
| Beispiel | 23 | = | 2-TMCPamino-4-3'ethoxy-propylamino-6-methylthio-s-triazin |
| Beispiel | 24 | = | 2-TMCPamino-4-3'butoxy-propylamino-6-methylthio-s-triazin |
| Beispiel | 25 | = | 2-TMCPamino-4-3'hexoxy-propylamino-6-methylthio-s-triazin |
| Beispiel | 26 | = | 2-TMCPamino-4-3'isopropoxy-propylamino-6-methylthio-s-triazin |
| Beispiel | 27 | = | 2-TMCamino-4-amino-6-methylthio-s-triazin |
| Beispiel | 28 | = | 2-TMCamino-4-methylamino-6-methylthio-s-triazin |
| Beispiel | 29 | = | 2-TMCamino-4-ethylamino-6-methylthio-s-triazin |
| Beispiel | 30 | = | 2-TMCamino-4-isopropylamino-6-methylthio-s-triazin |
| Beispiel | 31 | = | 2-TMCamino-4-propylamino-6-methylthio-s-triazin |
| Beispiel | 32 | = | 2-TMCamino-4-tert.-butylamino-6-methylthio-s-triazin |

Beispiel 33. = 2-TMCamino-4-2'methoxy-ethylamino-6-methylthio-s-triazin

Beispiel 34 = 2-TMCamino-4-3'methoxy-propylamino-6-methylthio-s-triazin

Beispiel 35 = 2-TMCamino-4-3'ethoxy-propylamino-6-methylthio-s-triazin

Beispiel 36 = 2-TMCamino-4-3'butoxy-propylamino-6-methylthio-s-triazin

Tabelle 1 b

Liste der hergestellten Verbindungen mit ihren verschiedenen Substituenten und sonstigen Kenngrößen gemäß Formel I und ihren Schmelzbereichen

| Beispiel Nr. | $R_1$ | A | n | Stellung von $R_n$ | Schmelzbereich °C |
|---|---|---|---|---|---|
| 1 | $-H$ | $-CH_2-$ | 0 | --- | 65 - 73 |
| 2 | $-CH_3$ | $-CH_2-$ | 0 | --- | 90 - 95 |
| 3 | $-CH_2-CH_3$ | $-CH_2-$ | 0 | --- | 83 - 88 |
| 4 | $-CH=(CH_3)_2$ | $-CH_2-$ | 0 | --- | 120 - 123 |
| 5 | $-CH_2-CH_2-CH_3$ | $-CH_2-$ | 0 | --- | 76 - 82 |
| 6 | $-C(CH_3)_3$ | $-CH_2-$ | 0 | --- | 105 - 110 |
| 7 | $-CH_2-CH_2OH$ | $-CH_2-$ | 0 | --- | 35 - 45 |
| 8 | $-CH_2-CH_2-CH_2-OCH_3$ | $-CH_2-$ | 0 | --- | 65 - 70 |
| 9 | $-CH_2-CH_2-CH_2-OCH_2-CH_3$ | $-CH_2-$ | 0 | --- | 60 - 66 |
| 10 | $-CH_2-CH_2OCH_3$ | $-CH_2-$ | 0 | --- | 85 - 97 |
| 11 | $-CH_2-CH_2-CH_2-OCH_2-CH_2-CH_2-CH_3$ | $-CH_2-$ | 0 | --- | 20 - 28 |
| 12 | $-CH_2-CH_2-CH_2-OCH_2-CH_2-CH_2-CH_2-CH_2-CH_3$ | $-CH_2-$ | 0 | --- | 20 - 30 |
| 13 | $-CH_2-CH_2-CH_2-O-CH=(CH_3)_2$ | $-CH_2-$ | 0 | --- | 25 - 30 |
| 14 | $-CH_2-CH_2-CH_2-O-CH_2-CH=(CH_3)_2$ | $-CH_2-$ | 0 | --- | 25 - 32 |
| 15 | $-H$ | $-CH_2-$ | 3 | 2,2,4(2,4,4) | 50 - 57 |

Fortsetzung von Tabelle 1 b

| Beispiel Nr. | $R_1$ | A | n | Stellung von $R_n$ | Schmelzbereich °C |
|---|---|---|---|---|---|
| 16 | $-CH_3$ | $-CH_2-$ | 3 | 2,2,4(2,4,4) | 52 - 63 |
| 17 | $-CH_2-CH_3$ | $-CH_2-$ | 3 | 2,2,4(2,4,4) | 54 - 60 |
| 18 | $-CH=(CH_3)_2$ | $-CH_2-$ | 3 | 2,2,4(2,4,4) | 30 - 38 |
| 19 | $-CH_2-CH_2-CH_3$ | $-CH_2-$ | 3 | 2,2,4(2,4,4) | 48 - 54 |
| 20 | $-C(CH_3)_3$ | $-CH_2-$ | 3 | 2,2,4 (2,4,4) | 28 - 34 |
| 21 | $-CH_2-CH_2-OCH_3$ | $-CH_2-$ | 3 | 2,2,4 (2,4,4) | 30 - 40 |
| 22 | $-CH_2-CH_2-CH_2OCH_3$ | $-CH_2-$ | 3 | 2,2,4 (2,4,4) | 42 - 46 |
| 23 | $-CH_2-CH_2-CH_2-OCH_2-CH_3$ | $-CH_2-$ | 3 | 2,2,4 (2,4,4) | 34 - 38 |
| 24 | $-CH_2-CH_2-CH_2OCH_2-CH_2-CH_2-CH_3$ | $-CH_2-$ | 3 | 2,2,4 (2,4,4) | 30 - 35 |
| 25 | $-CH_2-CH_2-CH_2-OCH_2-CH_2-CH_2-CH_2-CH_2-CH_3$ | $-CH_2-$ | 3 | 2,2,4 (2,4,4) | 26 - 32 |
| 26 | $-CH_2-CH_2-CH_2-O-CH=(CH_3)_2$ | $-CH_2-$ | 3 | 2,2,4 (2,4,4) | 25 - 30 |
| 27 | $-H$ | $-CH_2-CH_2-$ | 3 | 3,3,5 (cis-trans-Gemisch) | 55 - 60 |
| 28 | $-CH_3$ | $-CH_2-CH_2-$ | 3 | 3,3,5 " | 55 - 62 |
| 29 | $-CH_2-CH_3$ | $-CH_2-CH_2-$ | 3 | 3,3,5 " | 53 - 58 |
| 30 | $-CH=(CH_3)_2$ | $-CH_2-CH_2-$ | 3 | 3,3,5 " | 50 - 54 |

Fortsetzung von Tabelle 1 b

| Beispiel Nr. | $R_1$ | A | n | Stellung von $R_n$ | Schmelzbereich °C |
|---|---|---|---|---|---|
| 31 | $-CH_2-CH_2-CH_3$ | $-CH_2-CH_2-$ | 3 | 3,3,5 (cis-trans-Gemisch) | 38 - 44 |
| 32 | $-C\equiv(CH_3)_3$ | $-CH_2-CH_2-$ | 3 | 3,3,5 " | 42 - 46 |
| 33 | $-CH_2-CH_2-O-CH_3$ | $-CH_2-CH_2-$ | 3 | 3,3,5 " | 44 - 53 |
| 34 | $-CH_2-CH_2-CH_2-O-CH_3$ | $-CH_2-CH_2-$ | 3 | 3,3,5 " | 43 - 48 |
| 35 | $-CH_2-CH_2-CH_2-O-CH_2-CH_3$ | $-CH_2-CH_2-$ | 3 | 3,3,5 " | 40 - 45 |
| 36 | $-CH_2-CH_2-CH_2-O-CH_2-CH_2-CH_2-CH_3$ | $-CH_2-CH_2-$ | 3 | 3,3,5 " | 26 - 32 |

Verwendung und Einsatz-Möglichkeiten der neuen Verbindungen

Die erfindungsgemäßen Wirkstoffe können sowohl einzeln verwendet werden, als auch - bei verschiedenen Substituenten $R_1$ und/oder R, verschiedenen Bedeutungen von A oder verschiedenen Werten von n, sowie verschiedenen Substitutionsstellen der n R-Substituenten in Mischung miteinander. Selbstverständlich kann man auch eine oder mehrere der genannten Wirkstoffe mit anderen Herbiziden, Fungiziden, Insektiziden, Wachstumsregulatoren usw. mischen und die jeweiligen Kulturpflanzen damit beaufschlagen.

Außerdem ist auch ein Zumischen zu Mineraldüngern möglich, am einfachsten zu Düngemittellösungen.

Üblicherweise werden den erfindungsgemäßen Wirkstoffen - gegebenenfalls in den oben erwähnten Mischungen - vor der Applikation Hilfsmittel aus der Gruppe der Träger-, Verdünnungs-Lösungs-, Netz-, Haft-, Dispergier- und/oder Emulgiermittel usw. zugesetzt und dadurch das Verspritzen, bzw. das Ermöglichen einer gleichmäßigen Verteilung der Wirkstoffe verbessert.

Bei den Verbindungen der Formel I handelt es sich um Herbizide, welche sowohl im Vor- als auch im Nachauflaufverfahren ein breites Selektivitätsspektrum gegenüber zahlreichen Kulturpflanzen und eine sehr gute Wirkung gegen Schadgräser und zweikeimblättrige Unkräuter aufweisen. Die vorliegenden Verbindungen sind im Vorauflaufverfahren und/oder Nachauflaufverfahren u.a. selektiv in Soja (17*), Baumwolle (4), Reis (15), Gerste (8) und Weizen (22).

* Botanische Namen s. Tabelle 2 Seite 14.
(Die Pflanzenarten sind in dieser Tabelle nach dem Alphabet der deutschen Namen angeordnet. Die Nummer

## Tabelle 2

### Botanische Namen der Testpflanzen:

| | deutsche Bezeichnung | | lateinische Bezeichnung |
|---|---|---|---|
| 1. | Ackerfuchsschwanz | = | Alopecurus myosuroides |
| 2. | Ackersenf | = | Sinapis alba |
| 3. | Amaranth | = | Amaranthus retroflexus |
| 4. | Baumwolle | = | Gossypium hirsutum |
| 5. | Floh-Knöterich | = | Polygonum persicaria |
| 6. | Flughafer | = | Avena fatua |
| 7. | Franzosenkraut | = | Galinsoga parviflora |
| 8. | Gerste | = | Hordeum vulgare |
| 9. | Hafer | = | Avena sativa |
| 10. | Hühnerhirse | = | Echinocloa crus-galli |
| 11. | Kamille | = | Matricaria maritima |
| 12. | Klatschmohn | = | Papaver rhoeas |
| 13. | Klettenlabkraut | = | Galium aparine |
| 14. | Mais | = | Zea mays |
| 15. | Reis | = | Oryza sativa |
| 16. | Saatwucherblume | = | Chrysanthenum segetum |
| 17. | Soja | = | Glycine max |
| 18. | Taubnessel | = | Lamium purpureum |
| 19. | Tomate | = | Lycopersicum esculentum |
| 20. | Vogelmiere | = | Stellaria media |
| 21. | Weißer Gänsefuß | = | Chenopodium alba |
| 22. | Weizen | = | Triticum aestivum |
| 23. | Wicke | = | Vicia angustifolia |
| 24. | Windhalm | = | Apera spica-venti |
| 25. | Winterraps | = | Brassica napus |
| 26. | Zuckerrübe | = | Beta vulgaris |

* Fortsetzung der Anmerkung * von Seite 13
hinter den deutschen Namen der vorliegenden Aufzählung auf den Seiten 13 und 15 erleichtert die Auffindung der betreffenden Pflanzennamen.)

Die erfindungsgemäßen Verbindungen weisen zudem ein breites Wirkungsspektrum gegen Schadgräser wie z.B. Ackerfuchsschwanz (1), Windhalm (24), Flughafer (6) und Hirsearten (Echinochloa spp., Digitaris spp., Setaria spp. Vgl. 10 ) auf. Eine Reihe von zweikeimblättrigen Unkräutern wird ebenfalls erfaßt, so u.a. Vogelmiere (20), Klettenlabkraut (13), Kamille (11), Weißer Gänsefuß (21), Ackersenf (2), Taubnessel (18), Amaranth (3), Klatschmohn (12), Knöterich (5) und Franzosenkraut (7).

Die Wirkstoffe können in einer Menge von 0,125 bis 10 kg/ha, vorzugsweise von 0,5 - 2,5 kg/ha, auf die Kulturen aufgebracht werden.

Die erfindungsgemäßen Verbindungen wirken befriedigend mindestens ab 0,25 kg/ha, berechnet auf reinen Wirkstoff, und können trotz ihres breiten Wirkungsspektrums in den oben genannten Kulturen ohne sichtbare Schädigung der Kulturpflanzen eingesetzt werden.

Die Verwendung der erfindungsgemäßen Verbindungen ist nicht nur auf die obengenannten Pflanzenarten beschränkt, sondern bezieht sich in gleicher Weise auch auf andere Pflanzen. In Abhängigkeit von der Konzentration können die Verbindungen auch zur Totalunkrautbekämpfung, z.B. auf Industrie- und Gleisanlagen oder auf Wegen und Plätzen mit und ohne Baumbewuchs, eingesetzt werden.

Auch zur Unkrautbekämpfung in Dauerkulturen wie z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen sind die erfindungsgemäßen Verbindungen geeignet.

Bei einigen Pflanzenarten wirken mehrere erfindungsgemäße Verbindungen hemmend auf das Längenwachstum, ohne jedoch zu Ertragsminderungen zu führen; sie können deshalb auch zusätzlich als wachstumsregulierendes Mittel eingesetzt werden. Einige der neuen Verbindungen können auch als Defoliants, Desiccants, Krautabtötungsmittel und Keimhemmungsmittel verwendet werden.

## Biologische Prüfung der Wirkstoffe

### A Gewächshaus-Versuchsreihe mit 4 kg/ha

Für sämtliche Verbindungen der Tabelle 1 a (bzw. 1 b) wurden mit einer Aufwandmenge von 4 kg/ha Gewächshausversuche mit der üblichen Auswahl von Nutz- und Schadpflanzen im Vor- und Nachauflauf durchgeführt. Dabei wurde im allgemeinen eine gute Wirkung bei den Unkräutern und Schadgräsern, sowie keine oder nur eine geringe Wirkung bei den Nutzpflanzen beobachtet.

### B Gewächshaus-Versuchsreihe mit 4,3,2 und 1 kg/ha

Für eine Auswahl der bei der ersten Versuchsreihe als optimal-wirkend festgestellten erfindungsgemäßen Verbindungen wurden mit einer Aufwandmenge von 4,3,2 und 1 kg/ha, bezogen auf reinen Wirkstoff,

die Gewächshaus-Versuche wiederholt und in den Tabellen 3 a und b zusammengestellt.

Die jeweils benötigten Wirkstoffmengen wurden dabei in 1000 l Wasser/ha suspendiert.

Im Vorauflauf erfolgte die Applikation auf die Oberfläche vor dem Keimen der Samen und

im Nachauflauf auf die Blattoberfläche im zweiten bis dritten Blattstadium.

Drei Wochen nach der Behandlung ist bei den erfindungsgemäßen Verbindungen eine sehr gute Wirkung gegen zweikeimblättrige und einkeimblättrige Pflanzen sowohl im Vor- als auch im Nachauflauf (Tabellen 3 a und 3 b) zu beobachten.

Die Auswertung der Versuchsergebnisse erfolgte durch die übliche Benotung, bei der
1 = volle Wirkung = Abtötung der Pflanzen und
5 = keine Wirkung = Pflanzen wie unbehandelt
bedeuten.

Die erfindungsgemäßen Verbindungen zeigen sowohl im Vor- als auch im Nachauflauf (Tabellen 3 a und 3 b) eine hervorragende Wirkung gegen Schadgräser (z.B. Ackerfuchsschwanz, Hirsearten, Windhalm) und zweikeimblättrige Unkräuter. Neben diesem breiten Wirkungsspektrum fällt aber die für Triazin-Verbindungen überraschende außerordentlich hohe Verträglichkeit in Getreidekulturen (wie Gerste, Weizen), Soja, Baumwolle und Reis u.a. im Vorauflauf auf, die die Vergleichsverbindungen nicht aufweisen.

Tabelle 3a Herbizide Wirkung der erfindungsgemäßen Verbindungen in den Aufwandmengen von 4, 3, 2 und 1 kg Wirkstoff/ha. Applikation vor dem Auflaufen der Pflanzen (Teil 1)

| Testpflanzen kg/ha | Beispiel 1 | | | | Beispiel 2 | | | | Beispiel 3 | | | | Beispiel 4 | | | | Beispiel 8 | | | | Beispiel 9 | | | | Beispiel 17 | | | |
|---|---|---|---|---|---|---|---|---|---|---|---|---|---|---|---|---|---|---|---|---|---|---|---|---|---|---|---|---|
| | 4 | 3 | 2 | 1 | 4 | 3 | 2 | 1 | 4 | 3 | 2 | 1 | 4 | 3 | 2 | 1 | 4 | 3 | 2 | 1 | 4 | 3 | 2 | 1 | 4 | 3 | 2 | 1 |
| Ackersenf | 4 | 5 | 5 | 5 | 1 | 3 | 4 | 5 | 1 | 3 | 4 | 4 | 5 | 5 | 5 | 5 | 1 | 1 | 1 | 4 | 4 | 4 | 5 | 5 | 3 | 3 | 5 | 5 |
| Tomate | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Hühnerhirse | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Hafer | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Flughafer | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Ackerfuchsschwanz | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Vogelmiere | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Klettenlabkraut | 1 | 1 | 2 | 3 | 1 | 1 | 2 | 3 | 1 | 1 | 2 | 2 | 4 | 4 | 4 | 5 | 4 | 4 | 4 | 5 | 4 | 4 | 4 | 5 | 3 | 4 | 4 | 5 |
| Kamille | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Weißer Gänsefuß | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 3 |
| Windhalm | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Gerste | 4 | 4 | 5 | 5 | 4 | 5 | 5 | 5 | 4 | 5 | 5 | 5 | 4 | 5 | 5 | 5 | 4 | 4 | 5 | 5 | 4 | 4 | 5 | 5 | 3 | 4 | 5 | 5 |
| Weizen | 4 | 4 | 5 | 5 | 4 | 5 | 5 | 5 | 4 | 5 | 5 | 5 | 4 | 5 | 5 | 5 | 4 | 4 | 5 | 5 | 4 | 4 | 4 | 5 | 3 | 4 | 5 | 5 |

Tabelle 7a Herbizide Wirkung der erfindungsgemäßen Verbindungen in den Aufwandmengen von 4, 3, 2 und 1 kg Wirkstoff/ha. Applikation vor dem Auflaufen der Pflanzen (Teil 2)

| Testpflanzen kg/ha | Beispiel 1 | | | | Beispiel 2 | | | | Beispiel 3 | | | | Beispiel 4 | | | | Beispiel 8 | | | | Beispiel 9 | | | | Beispiel 17 | | | |
|---|---|---|---|---|---|---|---|---|---|---|---|---|---|---|---|---|---|---|---|---|---|---|---|---|---|---|---|---|
| | 4 | 3 | 2 | 1 | 4 | 3 | 2 | 1 | 4 | 3 | 2 | 1 | 4 | 3 | 2 | 1 | 4 | 3 | 2 | 1 | 4 | 3 | 2 | 1 | 4 | 3 | 2 | 1 |
| Taubnessel | 1 | 1 | 1 | 1 | 1 | 1 | 1 | 1 | 1 | 1 | 1 | 1 | 1 | 1 | 1 | 1 | 1 | 1 | 1 | 4 | 1 | 1 | 3 | 4 | 1 | 1 | 2 | 4 |
| Amaranth | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 3 |
| Winterraps | 1 | 1 | 4 | 4 | 2 | 2 | 4 | 4 | 1 | 1 | 2 | 5 | 1 | 1 | 1 | 1 | 1 | 1 | 1 | 2 | 2 | 2 | 2 | 2 | 4 | 4 | 4 | 5 |
| Floh-Knöterich | 1 | 1 | 1 | 1 | 1 | 1 | 1 | 1 | 1 | 1 | 1 | 1 | 1 | 1 | 1 | 2 | 1 | 1 | 1 | 1 | 1 | 1 | 1 | 1 | 1 | 1 | 1 | 3 |
| Klatschmohn | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Borstenhirse | 1 | 1 | 1 | 1 | 1 | 1 | 1 | 1 | 1 | 1 | 1 | 1 | 1 | 1 | 1 | 4 | 1 | 1 | 1 | 1 | 1 | 1 | 1 | 1 | 1 | 1 | 1 | 2 |
| Bluthirse | 1 | 1 | 1 | 1 | 1 | 1 | 1 | 1 | 1 | 1 | 1 | 1 | 1 | 1 | 1 | 3 | 1 | 1 | 1 | 1 | 1 | 1 | 1 | 1 | 1 | 1 | 1 | 1 |
| Sesbania spp. | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Ipomoea spp. | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 3 |
| Soja | 3 | 3 | 3 | 5 | 3 | 3 | 4 | 4 | 4 | 4 | 4 | 5 | 4 | 5 | 5 | 5 | 4 | 4 | 4 | 5 | 4 | 5 | 5 | 5 | 5 | 5 | 5 | 5 |
| Baumwolle | 5 | 5 | 5 | 5 | 5 | 5 | 5 | 5 | 4 | 5 | 5 | 5 | 5 | 5 | 5 | 5 | 4 | 4 | 5 | 5 | 4 | 4 | 5 | 5 | 5 | 5 | 5 | 5 |
| Reis | 5 | 5 | 5 | 5 | 4 | 4 | 4 | 4 | 5 | 5 | 5 | 5 | 5 | 5 | 5 | 5 | 5 | 5 | 5 | 5 | 5 | 5 | 5 | 5 | 5 | 5 | 5 | 5 |
| Mais | 3 | 3 | 4 | 4 | 3 | 4 | 4 | 4 | 3 | 4 | 4 | 5 | 4 | 4 | 4 | 4 | 3 | 3 | 4 | 5 | 3 | 3 | 4 | 4 | 4 | 4 | 5 | 5 |

**Tabelle 3b** Herbizide Wirkung der erfindungsgemäßen Verbindungen in den Aufwandmengen von 4, 3, 2 und 1 kg Wirkstoff/ha. Applikation nach dem Auflaufen der Pflanzen (Teil 1)

| Testpflanzen | Beispiel 1 | | | | Beispiel 2 | | | | Beispiel 3 | | | | Beispiel 4 | | | | Beispiel 8 | | | | Beispiel 9 | | | | Beispiel 17 | | | |
|---|---|---|---|---|---|---|---|---|---|---|---|---|---|---|---|---|---|---|---|---|---|---|---|---|---|---|---|---|
| kg/ha | 4 | 3 | 2 | 1 | 4 | 3 | 2 | 1 | 4 | 3 | 2 | 1 | 4 | 3 | 2 | 1 | 4 | 3 | 2 | 1 | 4 | 3 | 2 | 1 | 4 | 3 | 2 | 1 |
| Ackersenf | 1 | 1 | 1 | 3 | 1 | 2 | 2 | 4 | 1 | 1 | 1 | 1 | 1 | 1 | 1 | 2 | 1 | 1 | 1 | 1 | 1 | 1 | 1 | 1 | 1 | 1 | 1 | 1 |
| Tomate | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Hühnerhirse | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Hafer | 1 | 1 | 1 | 1 | 1 | 1 | 1 | 1 | 1 | 1 | 1 | 1 | 1 | 1 | 3 | 3 | 1 | 1 | 1 | 1 | 1 | 1 | 1 | 1 | 1 | 1 | 1 | 1 |
| Flughafer | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Ackerfuchsschwanz | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Vogelmiere | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Klettenlabkraut | 1 | 1 | 1 | 2 | 1 | 1 | 2 | 3 | 1 | 1 | 1 | 2 | 5 | 5 | 5 | 5 | 1 | 1 | 2 | 4 | 1 | 1 | 3 | 3 | 1 | 1 | 3 | 3 |
| Kamille | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Weißer Gänsefuß | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Windhalm | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Gerste | 1 | 1 | 1 | 1 | 1 | 1 | 1 | 3 | 1 | 1 | 2 | 2 | 1 | 1 | 2 | 2 | 1 | 1 | 1 | 1 | 1 | 1 | 1 | 1 | 1 | 1 | 1 | 1 |
| Weizen | 1 | 1 | 1 | 1 | 1 | 1 | 1 | 2 | 1 | 1 | 2 | 3 | 3 | 3 | 3 | 3 | 1 | 1 | 1 | 1 | 1 | 1 | 1 | 1 | 1 | 1 | 1 | 1 |

**Tabelle 3b** Herbizide Wirkung der erfindungsgemäßen Verbindungen in den Aufwandmengen von 4, 3, 2 und 1 kg Wirkstoff/ha. Applikation nach dem Auflaufen der Pflanzen (Teil 2)

| Testpflanzen | Beispiel 1 | | | | Beispiel 2 | | | | Beispiel 3 | | | | Beispiel 4 | | | | Beispiel 8 | | | | Beispiel 9 | | | | Beispiel 17 | | | |
|---|---|---|---|---|---|---|---|---|---|---|---|---|---|---|---|---|---|---|---|---|---|---|---|---|---|---|---|---|
| kg/ha | 4 | 3 | 2 | 1 | 4 | 3 | 2 | 1 | 4 | 3 | 2 | 1 | 4 | 3 | 2 | 1 | 4 | 3 | 2 | 1 | 4 | 3 | 2 | 1 | 4 | 3 | 2 | 1 |
| Taubnessel | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Amaranth | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Winterraps | 1 | 1 | 1 | 1 | 1 | 1 | 1 | 1 | 1 | 1 | 1 | 1 | 1 | 1 | 1 | 1 | 1 | 1 | 1 | 2 | 1 | 1 | 1 | 1 | 1 | 1 | 1 | 1 |
| Floh-Knöterich | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Klatschmohn | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Borstenhirse | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Bluthirse | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Sesbania spp. | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Ipomoea spp. | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Soja | 1 | 1 | 1 | 1 | 1 | 1 | 1 | 1 | 1 | 1 | 1 | 1 | 1 | 2 | 3 | 3 | 1 | 1 | 3 | 4 | 2 | 2 | 3 | 3 | 2 | 2 | 3 | 3 |
| Baumwolle | 1 | 2 | 3 | 3 | 1 | 3 | 3 | 3 | 1 | 1 | 2 | 3 | 3 | 4 | 4 | 4 | 3 | 3 | 4 | 4 | 3 | 3 | 3 | 4 | 3 | 4 | 4 | 5 |
| Reis | 3 | 4 | 4 | 5 | 4 | 4 | 4 | 5 | 4 | 4 | 4 | 5 | 5 | 5 | 5 | 5 | 5 | 5 | 5 | 5 | 5 | 5 | 5 | 5 | 5 | 5 | 5 | 5 |
| Mais | 1 | 1 | 3 | 3 | 4 | 4 | 4 | 4 | 4 | 4 | 4 | 5 | 4 | 4 | 4 | 4 | 3 | 4 | 4 | 4 | 4 | 4 | 4 | 5 | 4 | 4 | 5 | 5 |

Die Verwendung einer erfindungsgemäßen Verbindung als Bodenherbizid in Getreidekulturen hat neben der spezifischen Wirkung gegen Schadgräser zudem den Vorteil, daß im Gegensatz zu den meisten bisher bekannten Bodenherbiziden in Getreide gleichzeitig ein besonders weites Spektrum an zweikeimblättrigen Unkräutern erfaßt wird, gleichzeitig verbunden mit einer sehr guten Wirkung gegen Ackerfuchsschwanz und eine Reihe zweikeimblättriger Unkräuter.

Aus den Tabellen 3 a und 3 b geht darüberhinaus auch die gute Selektivität der erfindungsgemäßen Verbindungen in einigen wichtigen anderen Kulturen (u.a. Baumwolle) im Vor- als auch im Nachauflauf hervor.

C. Gewächshausversuchsreihe mit 0,5 , 0,25 und 0,125 kg/ha

Wegen der vorhandenen hohen herbiziden Wirkung der erfindungsgemäßen Verbindungen wurden einige zusätzlich in noch niedrigeren Aufwandmengen (0,5, 0,25 und 0,125 kg Wirkstoff/ha) geprüft und mit zwei Vergleichsmitteln aus der Gruppe der s-Triazine verglichen.

Die dabei erhaltene Tabelle 4 zeigt, daß die erfindungsgemäßen Verbindungen in wichtigen Indikationen (u.a. Ackerfuchsschwanz, Windhalm, Flughafer, Vogelmiere, Taubnessel) die Vergleichsmittel in der herbiziden Wirkung übertreffen und darüberhinaus gleichzeitig eine hervorragende Kulturverträglichkeit (u.a. in Getreide) offenbaren.

In den niedrigen Konzentrationen ist zudem im Vorauflauf - bei gegebener Wirkung gegen Unkräuter und Schadgräser - eine Selektivität in Winterraps vorhanden.

Generell ist festzustellen, daß durch die erfindungsgemäßen Verbindungen die herbizide Wirkung des Vergleichsmittels 1 (Atrazin) z.B. gegen Ackerfuchsschwanz, Windhalm, Flughafer, Vogelmiere, Kamille und Taubnessel erreicht oder sogar übertroffen wird, daß andererseits aber - besonders im Vorauflaufverfahren - im Gegensatz zum Vergleichsmittel 1- eine hervorragende Selektivität in Getreide, vor allem in Gerste und in Weizen, erzielt wird.

Außerdem weisen die erfindungsgemäßen Produkte

**Tabelle 4** Herbizide Wirkung der erfindungsgemäßen Verbindungen in den Aufwandmengen 0,5, 0,25 und 0,125 kg/ha vor dem Auflaufen (VA) bzw. nach dem Auflaufen (NA) der Testpflanzen

| Verbindung | kg Wirkstoff/ha | Ackerfuchsschwanz | | Windhalm | | Hühnerhirse | | Mais | | Vogelmiere | | Kamille | | Gerste | | Weizen | | Wi-Raps | |
|---|---|---|---|---|---|---|---|---|---|---|---|---|---|---|---|---|---|---|---|
| | | VA | NA | VA | NA | VA | NA | VA | NA | VA | NA | VA | NA | VA | NA | VA | NA | VA | NA |
| Beispiel 1 | 0,5 | 1 | 1 | 1 | 1 | 1 | 1 | 3 | 3 | 1 | 1 | 1 | 1 | 4 | 1 | 4 | 1 | 5 | 1 |
| | 0,25 | 1 | 1 | 1 | 1 | 2 | 1 | 5 | 3 | 1 | 1 | 1 | 1 | 4 | 1 | 4 | 1 | 5 | 2 |
| | 0,125 | 2 | 1 | 2 | 1 | 3 | 2 | 5 | 5 | 3 | 1 | 1 | 1 | 5 | 2 | 5 | 2 | 5 | 2 |
| Beispiel 2 | 0,5 | 1 | 1 | 1 | 1 | 1 | 1 | 5 | 5 | 1 | 1 | 1 | 1 | 4 | 1 | 4 | 1 | 4 | 1 |
| | 0,25 | 1 | 1 | 1 | 2 | 3 | 1 | 5 | 5 | 1 | 1 | 1 | 1 | 5 | 2 | 5 | 2 | 5 | 1 |
| | 0,125 | 2 | 1 | 3 | 3 | 5 | 1 | 5 | 5 | 3 | 1 | 1 | 1 | 5 | 2 | 5 | 2 | 5 | 1 |
| Beispiel 3 | 0,5 | 1 | 1 | 1 | 1 | 2 | 1 | 5 | 5 | 1 | 1 | 1 | 1 | 5 | 3 | 5 | 3 | 5 | 1 |
| | 0,25 | 2 | 1 | 2 | 1 | 4 | 1 | 5 | 5 | 1 | 1 | 1 | 1 | 5 | 3 | 5 | 3 | 5 | 1 |
| | 0,125 | 3 | 1 | 4 | 2 | 5 | 3 | 5 | 5 | 3 | 1 | 1 | 1 | 5 | 5 | 5 | 3 | 5 | 2 |
| Beispiel 4 | 0,5 | 1 | 1 | 1 | 1 | 1 | 1 | 5 | 5 | 1 | 1 | 1 | 1 | 5 | 3 | 5 | 3 | 4 | 1 |
| | 0,25 | 1 | 1 | 2 | 1 | 3 | 1 | 5 | 5 | 3 | 1 | 1 | 1 | 5 | 3 | 5 | 3 | 5 | 1 |
| | 0,125 | 2 | 1 | 3 | 2 | 4 | 2 | 5 | 5 | 4 | 1 | 1 | 1 | 5 | 4 | 5 | 5 | 5 | 2 |
| Beispiel 17 | 0,5 | 2 | 1 | 1 | 1 | 4 | 1 | 5 | 5 | 1 | 1 | 1 | 1 | 5 | 4 | 5 | 3 | 5 | 1 |
| | 0,25 | 2 | 1 | 2 | 2 | 4 | 2 | 5 | 5 | 2 | 1 | 1 | 1 | 5 | 4 | 5 | 3 | 5 | 1 |
| | 0,125 | 2 | 1 | 3 | 3 | 5 | 3 | 5 | 5 | 4 | 1 | 1 | 1 | 5 | 4 | 5 | 4 | 5 | 1 |
| Vergleichsmittel 1 | 0,5 | 1 | 1 | 1 | 1 | 2 | 1 | 5 | 5 | 1 | 1 | 1 | 1 | 1 | 1 | 1 | 1 | 3 | 1 |
| | 0,25 | 2 | 1 | 1 | 2 | 4 | 2 | 5 | 5 | 1 | 1 | 1 | 1 | 2 | 1 | 1 | 1 | 3 | 1 |
| | 0,125 | 2 | 1 | 1 | 3 | 4 | 3 | 5 | 5 | 1 | 2 | 1 | 1 | 3 | 2 | 2 | 2 | 3 | 1 |
| Vergleichsmittel 2 | 0,5 | 2 | 1 | 1 | 1 | 4 | 1 | 3 | 3 | 1 | 1 | 1 | 1 | 4 | 1 | 4 | 1 | 3 | 1 |
| | 0,25 | 2 | 1 | 2 | 2 | 4 | 1 | 4 | 3 | 1 | 1 | 1 | 1 | 4 | 1 | 4 | 1 | 3 | 1 |
| | 0,125 | 3 | 1 | 4 | 3 | 5 | 1 | 5 | 5 | 3 | 1 | 1 | 1 | 4 | 2 | 4 | 1 | 5 | 1 |

Vergleichsmittel 1 = Atrazin

Vergleichsmittel 2 = Terbutryn

eine besssere Wirkung gegen Hirsearten auf, die bekanntlich in einigen wichtigen Kulturen, wie Baumwolle und Sojabohne, als Schadgräser bedeutsam sind.
Die erfindungsgemäßen Verbindungen sind im Gegensatz zu dem Vergleichsmittel 1 in Baumwolle und Sojabohne selektiv.

Das Vergleichsmittel 2 (2-Methylthio-4-äthylamino-6-tert.-butylamino-1,3,5-triazin = Terbutryn) ist als konstitutionell ähnliches Vergleichsmittel zwar in Getreidearten, wie Gerste und Weizen selektiv, jedoch ist die Wirkung gegen in Getreide auftretende Schadgräser (z.B. Ackerfuchsschwanz, Windhalm) deutlich geringer als bei den erfindungsgemäßen Verbindungen. Zudem zeigt keines der Vergleichsmittel die von einer Reihe der erfindungsgemäßen Verbindungen in den unteren Aufwandmengen gezeigte Selektivität in Winterraps.

7473/40-ri

Patentansprüche

1. Verwendung von Methylthio-cyclopentylamin- bzw. Methylthiocyclohexylamin-s-triazinen der allgemeinen Formel

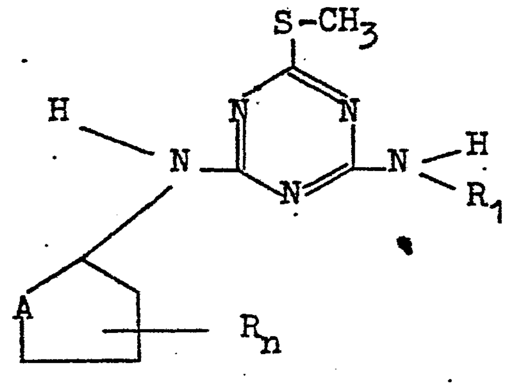

I,

in der

$R_1$ einen gerad- oder verzweigtkettigen Alkyl-, Alkoxy-, Alkoxyalkyl- oder Alkylol-Rest oder ein H-Atom,

R einen Rest aus der Gruppe der Alkyle mit ein bis drei C-Atomen und

A = $-CH_2-$ oder $-CH_2 - CH_2-$, bedeuten und

n für A = $-CH_2-$ jeweils 0 oder 1,2,3,4,5 oder 6 bzw. für
A = $-CH_2- CH_2-$ jeweils 3,4,5 oder 6

sein können,
wobei die Substituenten R an den Cycloalkylringen gleich oder verschieden sein können,

in geeigneter Applikationsform
zur selektiven Behandlung von Nutzpflanzenkulturen mit breitem Wirkungsspektrum bei Unkräutern und Schadgräsern

vor und/oder nach dem Auflaufen der Saat.

2. Verwendung von Mischungen von zwei oder mehreren Wirkstoffen nach Anspruch 1 gemäß Formel I, bei denen sich die Einzelwirkstoffe durch unterschiedliche Substituenten $R_1$ und/oder R und/oder von A und/oder die Werte von n voneinander unterscheiden.

3. Verwendung eines oder mehrerer der Wirkstoffe nach Anspruch 1 oder 2 in Mengen von je 0,05 bis 10 kg/ha, vorzugsweise von 0,5 bis 2,5 kg/ha.

4. Verwendung eines oder mehrerer der Wirkstoffe nach Anspruch 1 oder 2 und 3, dadurch gekennzeichnet, daß man die jeweilige Applikationsform durch Zumischen von Hilfsmitteln aus der Gruppe der Träger-, Verdünnungs-, Lösungs-, Netz-, Haft-, Dispergier- und Emulgiermittel gewinnt.

5. Verwendung eines oder mehrerer der Wirkstoffe nach Anspruch 1 und einem oder mehreren der Ansprüche 2 bis 4, gekennzeichnet durch die Zumischung von Stoffen aus der Gruppe der Herbizide, Fungizide, Insektizide, Wachstumsregulatoren usw..

6. Verwendung eines oder mehrerer der Wirkstoffe nach Anspruch 1 und gegebenenfalls nach einem oder mehreren der Ansprüche 2 bis 5 als Zusatz zu Mineraldüngern.

7473/40-ri